# EUROPEAN PATENT APPLICATION

(11) **EP 2 412 794 A1**
(43) Date of publication of application: **01.02.2012**
(21) Application number: 10171530.8
(22) Date of filing: 30.07.2010
(51) Int. Cl.: C12M 1/00, C12N 1/12

(54) **Method for circulatory cultivating photosynthetic microalgae**

(71) Applicant: Kairos Global Co., Ltd., Seoul 135-010 (KR); Eulgi University Industry Academy Corporation Foundation, Seongnam-si Gyeonggi-do 461-713 (KR)
(72) Inventor: Ki, Kwang Ho, Dangjin-gun, Chungcheongnam-do 343-882 (KR); Kang, Hee-Gyoo, Nowon-gu, Seoul 139-763, (KR); Kwon, Young II, Daejeon-si 302-845 (KR); Kim, Sun Jong, Anyang-si, Gyenggi-do 431-774 (KR); Lim, Hee Joung, Songpa-gu, Seoul 138-942, (KR); Kim, Mi Jeong, Seongnam-si, Gyeonggi-do 462.832, (KR)
(74) Representative: Tomlinson, Kerry John

(57) **Abstract**

A method for cultivating photosynthetic microalgae using a circulatory photobioreactor is provided. The circulatory photobioreactor comprises a first cultivating part (100), a second cultivating part (300) and a pump part (200) connecting the first cultivating part and the second cultivating part. The first cultivating part comprises a culture tank (101) in which culture media are supplied and a first light source coupled to the culture tank, which illuminates the inside of the culture tank. The second cultivating part comprises a culture pipe (330) placed outside of the culture tank and is supplied with cultures from the culture tank and a second light source (320) coupled to the culture pipe, which illuminates the inside of the culture pipe. The pump part is connected to both the first cultivating part and the second cultivating part in order to circulate the culture solution between them.

## Description

### BACKGROUND OF THE INVENTION

### 1. Field of the Invention

The following description relates to a method for circulatory cultivating photosynthetic microalgae.

### 2. Description of the Related Art

Currently, crops cultivated world-wide are used as major food sources for humans. However, yield per acre is not too much and the energy efficiency of solar power is extremely low. On the contrary, photosynthetic microalgae capable of growing underwater with solar energy, carbon dioxide and minute amounts of inorganic salts are favorable, since they can be cultivated in areas in which crops do not grow, can provide at least 20 times more protein per acre than conventional crops and can provide various useful substances and rare natural substances which are not produced in microorganisms, animals or plants. In particular, since algae whose cell size is large precipitate well, isolating them and extracting some substances therefrom is easy. In addition, they use solar energy as a major energy source, thus it is possible to use solar energy efficiently. Further, since they use carbon dioxide as a carbon source and have a photosynthetic system producing oxygen as byproducts, they can alleviate air pollution.

Accordingly, photosynthetic microalgae including genus *Chlorella,* genus *Dunaliella* and genus *Spirulina* has been studied, particularly microalgae belonging to genus *Spirulina* (hereinafter referred to as "Spirulina") since it is bigger than other photosynthetic microalgae, grows easily in an alkali-contaminated environment and can be applied in food, pharmaceutical and other industries. In the meanwhile, temperate regions such as Korea having climate characteristics such as occurrence of four seasons, dramatic variations of seasonal temperature and sunshine are developing indoor culture technology rather than outdoor culture technology.

For examples, Korean Patent Gazette No. 2004-0073693 discloses a method for cultivating Spirulina comprising controlling pH using charcoal as well as using the charcoal as a carbon source and Korean Patent Gazette No. 2006-0017033 discloses a media composition whose nitrogen and carbon concentrations are adjusted for maximized growth and harvest of Spirulina. In addition, Korean Patent Gazette No. 2002-0083558 discloses a high-density cultivating apparatus comprising a culture vessel having a cover on the top side thereof and a pH sensor and dispenser in the culture vessel, a frame for the culture vessel having fluorescent lights, a pH controller, an air pump and CO₂ culture tank and Korean Patent Gazette No. 2004-0019298 discloses an apparatus for cultivating microalgae comprising a culture vessel molded as a double cylinder-type consisting of an inner cylinder disposed horizontally and an outer cylinder, wherein at least the outer cylinder comprises a transparent material transmitting light and a gas inlet is opened at the bottom of the culture vessel. However, if charcoal is used, Spirulina is absorbed into the charcoal and the productivity is lowered thereby. If a new media composition is used, the price rate for the new media is higher than that of productivity, thus it could not be commercialized. As well, since Spirulina are photosynthetic bacteria, it is necessary to illuminate it at a sufficient amount of light. However, there are some problems such as that the efficiency of photosynthesis is lowered over time due to attachment of microalgae to the inner surface of the culture vessel and the light reaching the photosynthetic microalgae decreases thereby.

### SUMMARY OF THE INVENTION

It is possible to prevent contamination by various germs using the prior methods. However, the problems of excessive photosynthetic microalgae attachment to the inner surface of the culture vessels and the deteriorating cultivation efficiency have not been solved until now. If the problems are solved, it is possible to improve the cultivation efficiency of photosynthetic microalgae remarkably. However, there is no report to teach such a solution. Thus, the purpose of the present invention is to provide a novel method for cultivating microalgae capable of preventing attachment of photosynthetic microalgae to the inner surface of a culture vessel and thereby improving the cultivation efficiency.

The present inventors have solved the above-described problem and have confirmed that the area in which culture solution is exposed to light is maximized and growing photosynthetic microalgae do not attach to the inner surface of the culture vessels when the photosynthetic microalgae are cultivated using a tubular photobioreactor which further is equipped with a culture pipe and a pump to a conventional cylindrical photobioreactor.

In an aspect, the present invention provides a circulatory method for cultivating photosynthetic microalgae comprising the following:
a) injecting culture media in which photosynthetic microalgae is inoculated into a first cultivating part of a photobioreactor and performing a primary culture by illuminating the first cultivating part;
b) mixing culture solution after terminating the primary culture with fresh media added to the cultivating part and then transferring the mixed culture solution to a second cultivating part connected to the first cultivating part;
c) performing a further culture by illuminating the second cultivating part;
d) circulating further cultivated culture solution from the second cultivating part to the first cultivating part; and
e) recovering the culture solution and then filtering and harvesting photosynthetic microalgal bodies after terminating the cultivating.

In the method, the photosynthetic microalgae is preferably genus *Chlorella,* genus *Dunaliella* or genus *Spirulina,* and is more preferably genus *Spirulina,* but the invention is not limited thereto. The photosynthetic microalgae are injected through an inoculum inlet or a fresh media inlet with which the first cultivating part is equipped, but the invention is not limited thereto. In addition, the first cultivating part is equipped with a first light source for illuminating the first cultivating part. The light source may be coupled to the inside of the first cultivating part or be coupled to the outside of the first cultivating part. In the latter case, the wall of the first cultivating part is preferably composed of transparent materials or equipped with a transparent window through which light passes, but the invention is not limited thereto. The intensity of illumination of the primary culture is preferably about 4,000 to 8,000 lux, but the invention is not limited thereto. Cultivating temperature of the primary culture is preferably about 32 to 38 °C, but the invention is not limited thereto. An end of the second cultivating part is connected to a pump part connected to a first media outlet of the first cultivating part and the second cultivating part is preferably deformed as a curved pipe in a zigzag shape in order to maximize surface area of the second cultivating part, but the invention is not limited thereto. Intensity of illumination of the further culture is preferably about 4,000 to 8,000 lux, but the invention is not limited thereto and cultivating temperature of the further culture is preferably about 32 to 38°C, but the invention is not limited thereto. In a preferred embodiment, flow rate of the circulating culture solution in a culture pipe of the second cultivating part is controlled via a flow rate controller. The flow rate controller is preferably electronically coupled to the pump part or is built in the pump part. In a preferred embodiment, the flow rate is controlled between 5 to 50 cm/s. In a more preferred embodiment, the flow rate is 10 to 40 cm/s. In the most preferred embodiment, the flow rate is 20 to 30 cm/s.

In the meantime, pH of the primary culture and the further culture is preferably about 8.5 to 10, but the invention is not limited thereto.

In an embodiment of the present invention, the first cultivating part comprises a culture tank and transfer and circulation between the first cultivating part and the second cultivating part is performed by a pump, but the invention is not limited thereto. In this case, it is preferable to perform photosynthesis by photosynthetic microalgae by injecting mixed gases of carbon dioxide and nitrogen through the first cultivating part and to prevent contamination by various germs by maintaining positive pressure within the first cultivating part. The positive pressure is about 0.1 to 1.0 kg/cm²f (10-100 kPa) as used for conventional cultivation for photosynthetic microalgae, but the invention is not limited thereto.

In another aspect, the present invention provides a circulatory method for cultivating photosynthetic microalgae using a photobioreactor comprising a first cultivating part containing a culture tank, a second cultivating part whose shape is a tubular structure and a pump part connected between the first cultivating part and the second cultivating part which comprises the followings:
a) performing a primary culture by injecting culture media comprising an inoculum of photosynthetic microalgae to the first cultivating part and illuminating the first cultivating part;
b) mixing culture solution after terminating the primary culture with fresh media added to the cultivating part and circulating the mixed culture solution to the second cultivating part and to the first cultivating part, and performing a further culture by illuminating the second cultivating part; and
c) harvesting the photosynthetic microalgae by recovering and filtering the culture solution after the further culture.

In the method, the photosynthetic microalgae are preferably genus *Chlorella,* genus *Dunaliella* or genus *Spirulina,* and are more preferably genus *Spirulina,* but the invention is not limited thereto. The photosynthetic microalgae are injected through an inoculum inlet or a fresh media inlet with which the first cultivating part is equipped, but the invention is not limited thereto. In addition, the first cultivating part equips with a first light source for illuminating the first cultivating part. The light source may be coupled to the inside of the first cultivating part or be coupled to the outside of the first cultivating part. In the latter case, wall of the first cultivating part is preferably composed of transparent materials or equipped with a transparent window through which light passes, but the invention is not limited thereto. Intensity of illumination of the primary culture is preferably about 4,000 to 8,000 lux, but the invention is not limited thereto. Cultivating temperature of the primary culture is preferably about 32 to 38 °C, but the invention is not limited thereto. An end of the second cultivating part is connected to the pump part connected to a first media outlet of the first cultivating part and the second cultivating part is preferably deformed as a curved pipe in a zigzag shape in order to maximize surface area of the second cultivating part, but the invention is not limited thereto. Intensity of illumination of the further culture is preferably about 4,000 to 8,000 lux, but the invention is not limited thereto and cultivating temperature of the further culture is preferably about 32 to 38 °C, but the invention is not limited thereto. In a preferred embodiment, flow rate of the circulating culture solution in a culture pipe of the second cultivating part is controlled via a flow rate controller. The flow rate controller is preferably electronically coupled to the pump part or is built in the pump part. In a preferred embodiment, the flow rate is controlled between 5 to 50 cm/s. In a more preferred embodiment, the flow rate is 10 to 40 cm/s. In the most preferred embodiment, the flow rate is 20 to 30 cm/s.

In the meantime, pH of the primary culture and the further culture is preferably about 8.5 to 10, but the invention is not limited thereto.

In an embodiment of the present invention, the first cultivating part comprises a culture tank and transfer and circulation between the first cultivating part and the second cultivating part is performed by a pump, but the invention is not limited thereto. In this case, it is preferable to perform photosynthesis by photosynthetic microalgae by injecting mixed gases of carbon dioxide and nitrogen through the first cultivating part and to prevent contamination by various germs by maintaining positive pressure within the first cultivating part. The positive pressure is about 0.1 to 1.0 kg/cm²f (10-100 kPa) as used for conventional cultivation for photosynthetic microalgae, but the invention is not limited thereto.

The method for circulatory cultivating photosynthetic microalgae of the present invention prevents attachment of photosynthetic microalgae on the inner surface of culture vessel and can increase the culture efficiency of the photosynthetic microalgae thereby. Accordingly, one can cultivate photosynthetic microalgae economically.

### BRIEF DESCRIPTION OF THE DRAWINGS

The accompanying drawings, together with the specification, illustrate exemplary embodiments of the present invention, and, together with the description, serve to explain the principles of the present invention.

Fig. 1 is a schematic diagram showing a photobioreactor according to an embodiment of the present invention.

Fig. 2 is a sectional view of the first cultivating part included in the photobioreactor of the present invention.

Fig. 3 is a plan view illustrating an embodiment of the second cultivating part included in the tubular photobioreactor.

### DETAILED DESCRIPTION OF THE INVENTION

The present inventors performed various experiments in order to investigate the reason why cultivated photosynthetic microalgae attach on the inner surface of culture vessels. As a result, the two reasons are confirmed. The first one is recognized as the uncontrolled proliferation of microalgae along with the growth thereof according to applying periodical light condition and dark condition. The other is recognized that photosynthetic microalgae are attached on the site where flow velocity is low because the flow of culture solution is restricted locally and then the site of attachment is expanded.

In this regard, the present inventors tried to design a culture vessel capable of lowering proliferating rate of photosynthetic microalgae and securing proper flow of culture solution by applying only light conditions. However, the present inventors confirmed that it is impossible to obtain proper flow of culture solution even though raising stirring rate with a conventional cylindrical photobioreactor having an agitator.

Thus, the present inventors tried to run the culture solution containing photosynthetic microalgae using other methods rather than using only an agitator and confirmed that circulating the culture solution from the culture tank to a pipe whose two ends are coupled to the culture tank can make the culture solution flow well compared with the method using only an agitator. Accordingly, the present inventors came to invent a method for cultivating photosynthetic microalgae based on circulating culture solution externally using a tubular photobioreactor equipped with a culture pipe having a light source and a pump.

According to the method of the present invention which uses a circulatory photobioreactor comprising a culture pipe having a secondary light source and a pump connecting the culture pipe and the main body of the photobioreactor, which make it possible to circulate culture solution between the culture pipe and the main body, one can cultivate photosynthetic microalgae without permitting attachment of the photosynthetic microalgae to the inner surface of culture vessels by circulating the culture solution between the culture pipe and the main body at a constant rate, maximizing the area of the culture pipe to be illuminated and flowing the culture solution sufficiently thereby using the circulatory photobioreactor.

The method according to embodiments of the present invention is described in detail as follows:

In an embodiment of the present invention, a circulatory method for cultivating photosynthetic microalgae comprises the following:
a) injecting culture media in which photosynthetic microalgae are inoculated into a first cultivating part of a photobioreactor and performing a primary culture by illuminating the first cultivating part;
b) mixing culture solution after terminating the primary culture with fresh media added to the cultivating part and then transferring the mixed culture solution to a second cultivating part connected to the first cultivating part;
c) performing a further culture by illuminating the second cultivating part;
f) circulating further cultivated culture solution from the second cultivating part to the first cultivating part; and
g) recovering the culture solution and then filtering and harvesting photosynthetic microalgal bodies after terminating the cultivating.

In the method, the photosynthetic microalgae are preferably genus *Chlorella,* genus *Dunaliella* or genus *Spirulina,* and is more preferably genus *Spirulina,* but the invention is not limited thereto. The photosynthetic microalgae are injected through an inoculum inlet or a fresh media inlet with which the first cultivating part is equipped, but the invention is not limited thereto. In addition, the first cultivating part is equipped with a first light source for illuminating the first cultivating part. The light source may be coupled to the inside of the first cultivating part or be coupled to the outside of the first cultivating part. In the latter case, the wall of the first cultivating part is preferably composed of transparent materials or equipped with a transparent window through which light passes, but the invention is not limited thereto. The intensity of illumination of the primary culture is preferably about 4,000 to 8,000 lux, but the invention is not limited thereto. Cultivating temperature of the primary culture is preferably about 32 to 38 °C, but the invention is not limited thereto. An end of the second cultivating part is connected to the pump part connected to a first media outlet of the first cultivating part and the second cultivating part is preferably deformed as a curved pipe in a zigzag shape in order to maximize the surface area of the second cultivating part, but the invention is not limited thereto. The intensity of illumination of the further culture is preferably about 4,000 to 8,000 lux, but the invention is not limited thereto and cultivating temperature of the further culture is preferably about 32 to 38 °C, but the invention is not limited thereto.

In a preferred embodiment, the flow rate of the circulating culture solution in a culture pipe of the second cultivating part is controlled via a flow rate controller. The flow rate controller is preferably electronically coupled to the pump part or is built in the pump part. In a preferred embodiment, the flow rate is controlled between 5 to 50 cm/s. In a more preferred embodiment, the flow rate is 10 to 40 cm/s. In the most preferred embodiment, the flow rate is 20 to 30 cm/s. Control of the flow rate of culture solution is important for successful cultivation of Spirulina, because Siprulina is a multicellular spiral alga. If the flow rate is low, attachment of Spirulina occurs and gas exchange and illumination get worse due to inappropriate hydrodynamics. In contrast, high flow rate results in loss of useful materials in Spirulina. Thus, the flow rate should be controlled properly.

In the meantime, the pH of the primary culture and the further culture is preferably about 8.5 to 10, but the invention is not limited thereto.

In an embodiment of the present invention, the first cultivating part comprises a culture tank and transfer and circulation between the first cultivating part and the second cultivating part is performed by a pump, but the invention is not limited thereto. In this case, it is preferable to perform photosynthesis by photosynthetic microalgae by injecting mixed gases of carbon dioxide and nitrogen through the first cultivating part and to prevent contamination by various germs by maintaining positive pressure within the first cultivating part. The positive pressure is about 0.1 to 1.0 kg/cm²f (10-100 kPa) as used for conventional cultivation for photosynthetic microalgae, but the invention is not limited thereto.

In another embodiment of the present invention, a circulatory method for cultivating photosynthetic microalgae comprises the followings:
a) performing a primary culture by injecting culture media comprising an inoculum of photosynthetic microalgae to the first cultivating part and illuminating the first cultivating part;
b) mixing culture solution after terminating the primary culture with fresh media added to the cultivating part and circulating the mixed culture solution to the second cultivating part and to the first cultivating part, and performing a further culture by illuminating the second cultivating part; and
c) harvesting the photosynthetic microalgae by recovering and filtering the culture solution after the further culture.

In the method, the photosynthetic microalgae are preferably genus *Chlorella,* genus *Dunaliella* or genus *Spirulina,* and are more preferably genus *Spirulina,* but the invention is not limited thereto. The photosynthetic microalgae are injected through an inoculum inlet or a fresh media inlet with which the first cultivating part is equipped, but the invention is not limited thereto. In addition, the first cultivating part is equipped with a first light source for illuminating the first cultivating part. The light source may be coupled to the inside of the first cultivating part or be coupled to the outside of the first cultivating part. In the latter case, the wall of the first cultivating part is preferably composed of transparent materials or equipped with a transparent window through which light passes, but the invention is not limited thereto. Intensity of illumination of the primary culture is preferably about 4,000 to 8,000 lux, but the invention is not limited thereto. Cultivating temperature of the primary culture is preferably about 32 to 38 °C, but the invention is not limited thereto. An end of the second cultivating part is connected to the pump part connected to a first media outlet of the first cultivating part and the second cultivating part is preferably deformed as a curved pipe in a zigzag shape in order to maximize surface area of the second cultivating part, but the invention is not limited thereto. Intensity of illumination of the further culture is preferably about 4,000 to 8,000 lux, but the invention is not limited thereto and cultivating temperature of the further culture is preferably about 32 to 38 °C, but the invention is not limited thereto.

In the meantime, the pH of the primary culture and the further culture is preferably about 8.5 to 10, but the invention is not limited thereto.

In a preferred embodiment, flow rate of the circulating culture solution in a culture pipe of the second cultivating part is controlled via a flow rate controller. The flow rate controller is preferably electronically coupled to the pump part or is built in the pump part. In a preferred embodiment, the flow rate is controlled between 5 to 50 cm/s. In a more preferred embodiment, the flow rate is 10 to 40 cm/s. In the most preferred embodiment, the flow rate is 20 to 30 cm/s.

In an embodiment of the present invention, the first cultivating part comprises a culture tank and transfer and circulation between the first cultivating part and the second cultivating part is performed by a pump, but the invention is not limited thereto. In this case, it is preferable to perform photosynthesis by photosynthetic microalgae by injecting mixed gases of carbon dioxide and nitrogen through the first cultivating part and to prevent contamination by various germs by maintaining positive pressure within the first cultivating part. The positive pressure is about 0.1 to 1.0 kg/cm²f (10-100 kPa) as used for conventional cultivation for photosynthetic microalgae, but the invention is not limited thereto.

Hereinafter, the photobioreactor used for the circulatory method for cultivating photosynthetic microalgae is described in detail using the enclosed drawings.

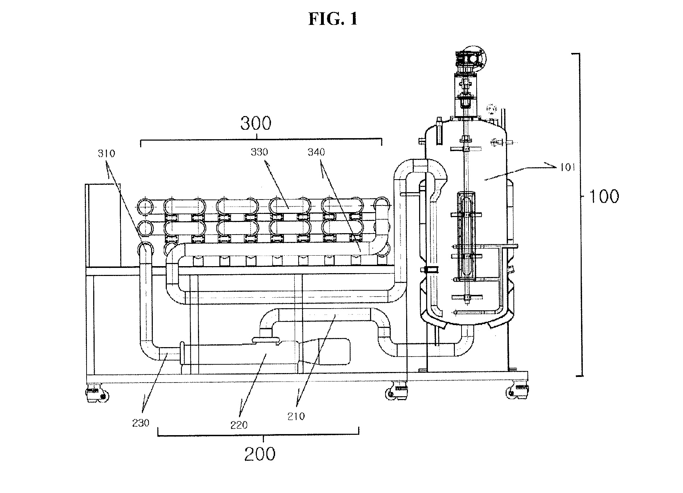

Fig. 1 is a schematic diagram showing a photobioreactor according to an embodiment of the present invention. As shown Fig. 1, the photobioreactor may comprise mainly a first cultivating part 100, a second cultivating part 300 and a pump part 200 connected to the first cultivating part and the second cultivating part so as to circulation of culture solution between the first cultivating part and the second cultivating part.

Since the first cultivating part 100, the pump part 200 and the second cultivating part 300 are connected to one another, they make it possible to cultivate photosynthetic microalgae by circulating culture solution in the following order: first cultivating part 100 → the pump part 200 → the second cultivating part 300 → the first cultivating part 100.

The second cultivating part 300 may be positioned apart from the first cultivating part 100. The first cultivating part 100 may be provided as a cylinder-type vessel as shown in Fig. 1, but the invention is not limited thereto. For example, the shape of the first cultivating part 100 can be of various forms such as a polygonal column. The second cultivating part 300 may be provided as a tubular shape and can include pipes molded with various shapes, etc.

The pump part 200 is connected to the first cultivating part 100 and the second cultivating part 300 in order that the culture solution can circulate between the first cultivating part 100 and the second cultivating part 300. For example, the pump part 200 can be positioned between the first cultivating part 100 and the second cultivating part 300 and can be equipped with a third culture solution inlet 210 connected to a first culture solution outlet 132 of the first cultivating part 100 and can be equipped with a third culture solution outlet 230 connected to a pump 220 and a second culture solution inlet 310 of the second cultivating part 300. Accordingly, the culture solution flowing from the first cultivating part 100 is transferred to the second cultivating part 300 and the culture solution circulates in the following order: the first cultivating part 100 → the pump part 200 → the second cultivating part 300 → the first cultivating part 100. The pump part 200 can control the flow rate of culture solution circulating through the second cultivating part 300 via a flow rate controller coupled electronically thereto. In a preferred embodiment, the flow rate controller is built in the pump part 200. Control of the flow rate of culture solution is important for successful cultivation of Spirulina, because Siprulina is a multicellular spiral alga. If the flow rate is low, attachment of Spirulina occurs and gas exchange and illumination get worse due to inappropriate hydrodynamics. In contrast, high flow rate results in loss of useful materials in Spirulina. Thus, the flow rate should be controlled properly. It is preferred to control the flow rate between 1 to 50 cm/s. More preferably, the flow rate is 10 to 40 cm/s. In the most preferred embodiment, the flow rate is 20 to 30 cm/s.

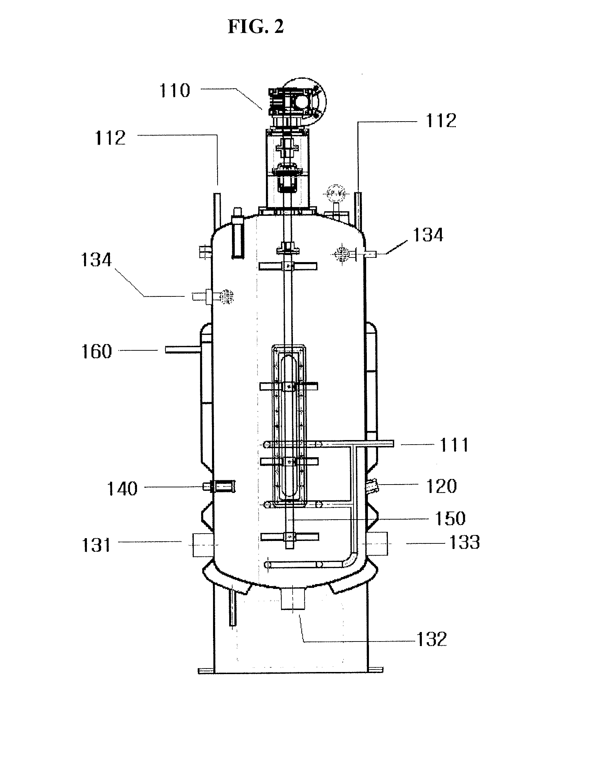

Fig. 2 is a sectional view of the first cultivating part 100 included in the photobioreactor of the present invention. As shown in Fig. 2, the first cultivating part 100 can be equipped with a cylindrical culture tank 101, an inoculum inlet 110, a gas inlet 111, a port for sensors 120, a first culture solution inlet 131, a first culture solution outlet 132, a final outlet 133, a pressure-controlling valve 112, a fresh media inlet 134, a first light source 140, an agitator 150 and a temperature controller 160.

For example, the inoculum inlet 110 may be coupled to the upper part of the culture tank 101, the gas inlet 111 and the port for sensors 120 may be coupled to the lower part of the culture tank 101. However, the arrangement is provided as an exemplary embodiment and may be varied according to changes of the shape of the culture tank 101.

For the primary culture, an inoculum may be injected via the inoculum inlet 110 or the fresh media inlet 134. However, it is preferable to use the inoculum inlet 110 in order to keep the environment sterile.

In the meantime, the gas inlet 111 may be provided so as to inject various gases such as nitrogen and carbon dioxide mixture gases to the inside of the culture tank 101. Accordingly, it is possible to prevent contamination by various germs originated from external environment during cultivating of photosynthetic microalgae by maintaining internal positive pressure in the culture tank 101. The positive pressure may be 0.1 to 1.0 kg/cm²f (10-100 kPa) as used for the conventional cultivation of microalgae, but the invention is not limited thereto.

The port for sensors 120 can be equipped with various sensors such as a pH sensor, a CO₂ sensor, a DO sensor and a temperature sensor.

In the meantime, the culture solution flows into the first cultivating part 100 from the second cultivating part through the first culture solution inlet 131 and flows out of the first cultivating part 100 to the pump part 200 via the first culture solution outlet 132. When the cultivation is finished, the final culture solution is released to the outside of the first cultivating part 100 through the final outlet 133.

For example, the pressure-controlling valve 112 which is a one-way valve opened by the pressure of oxygen generated by cultivating the photosynthetic microalgae may be composed to be opened to discharge gases externally when the internal pressure is maintained as positive, but to be closed to stop discharging gases when the internal pressure is lowered.

The fresh media inlet 134 is a stick-type tube whose end is globular shape, may be provided as a spray ball shape having a plentiful of micro nozzles on the surface of the globular end. Fresh media can be dispensed in a microspray into the first cultivating part 100 through the spray ball, thus the fresh media inlet 134 takes a role in eliminating foam generated in the first cultivating part 100. Further, the fresh media inlet 134 may be used for supplying detergents for washing the insides of the first cultivating part 100, the pump part 200 and the second cultivating part 300.

The first light source 140 which is a device for emitting light capable of facilitating photosynthesis during cultivation of photosynthetic microalgae emits light of three wavelengths or five wavelengths. In this regard, it is preferred that the intensity of illumination and the light-dark cycle are controlled automatically according to cultivation conditions. For example, the first light source 140 may be placed at the inside of the culture tank 101. In another embodiment, if the culture tank 100 comprises a transparent materials or parts of the culture tank 100 is equipped with a transparent window through which light passes, the light source 140 may be placed at the outside of the culture tank 101.

The agitator 150 may be coupled to the inner-lower part and takes a role in mixing the culture solution in the primary culture, and mixing the culture solution remaining in the first cultivating part 100 with culture solution flowing from the first culture solution inlet 131. The temperature controller 160 may be attached at the outside of the first cultivating part 100 and takes a role in controlling temperature. The temperature controller may be a water jacket capable of controlling culture temperature by circulating water with appropriate temperature through the jacket, but the invention is not limited thereto. Further, an observation window through which one can check the inside of the first cultivating part 100 may be installed additionally.

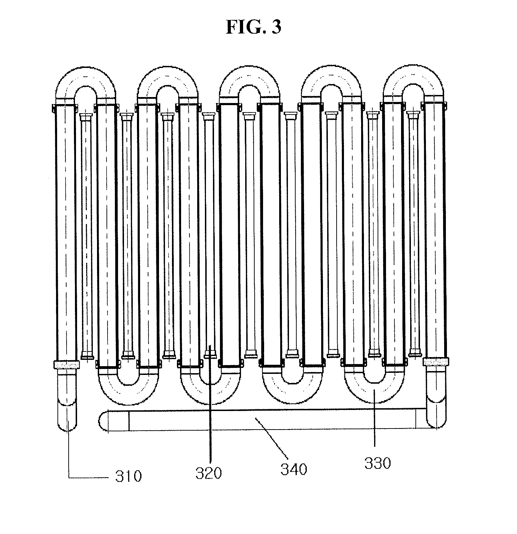

Fig. 3 is a plan view illustrating an embodiment of the second cultivating part 300 included in the tubular photobioreactor. As shown in Fig. 3, the second cultivating part 300 is equipped with a second culture solution inlet 310 connected to a third culture solution outlet 230 of the pump part 200, a culture pipe 330 and a second culture solution outlet 340 connected to the first culture solution inlet 131 of the first cultivating part 100.

The second light source 320 may be coupled to parts or whole of the second cultivating part 300. For example, the second light source 320 may be coupled to the outside of the culture pipe 330, extended longitudinally therethrough. The culture pipe 330 of the second cultivating part 300 plays a role in circulating the culture solution including photosynthetic microalgae and providing an environment where the circulating photosynthetic microalgae perform photosynthesis receiving light from the second light source 320. Thus, the culture pipe 330 may be composed of transparent materials in order to transmit the light emitted from the second light source 320. The whole culture pipe 330 may be composed of only transparent materials or only parts where light passes through are composed of transparent materials. Alternatively, the second light source 320 may be placed in the inside of the culture pipe 330. In this case, the second light source 310 is preferably coupled to the inner surface and is preferably an LED (light-emitting diode) and the culture pipe 330 may be composed of opaque materials. Additionally, sensors such as a pH sensor, a CO₂ sensor, a DO sensor, a temperature sensor may be attached at one or more sides of the culture pipe 330. Further, the culture pipe 330 is preferably deformed as a narrow and long tubular shape in order to maximize area exposed to light. In this case, the culture pipe 330 is preferably deformed as a folded structure and the second light source 320 may be provided multiply between the folded structures of the culture pipe 330.

In an embodiment of the present invention, the culture pipe 330 of the second cultivating part 300 may be composed as a parallel multiple folded form including straight parts and curved parts on one frame or such a folded form may be piled up as a multi-layer structure. Also, the frame which is a scaffold for anchoring the second light source 320 may additionally comprise a power supply for the second light source 320. In addition, the second light source 320 plays a role in emitting light for the photosynthesis by the photosynthetic microalgae during the cultivation. Light emitted from the second light source 320 is preferably three-wavelength or five-wavelength similar to daylight but the invention is not limited thereto and it is preferred that the intensity of illumination and the light-dark cycle are controlled automatically according to cultivation conditions.

In a preferred embodiment, the culture pipe 330 has an inside diameter of 3 to 30 cm. In a more preferred embodiment, the inside diameter is 5 to 20 cm. In the most preferred embodiment, the inside diameter is 10 to 15 cm. The inside diameter of the culture pipe is important for cultivating Spirulina. If the inside diameter is more than 30 cm, the productivity gets worse due to inappropriate illumination. In contrast, if the inside diameter is less than 5 cm, it is difficult to scale-up culture volume.

Hereinafter, the present invention is described more particularly through the following examples. The examples are only for explaining the present invention, thus those having ordinary skill in the art will understand that changes can be made to the specific examples without departing from the scope of the invention, which is defined by the claims.

### Comparative example 1: Cultivation of Spirulina using a prior high-density culture apparatus for microalgae

The present inventor cultivated Spirulina using a high-density culture apparatus for microalgae disclosed in Korean Patent Gazette No. 2002-0083558.

Particularly, the present inventors performed a primary culture for 3 days using the high-density culture apparatus at 35 °C under 6,000 lux of illumination in 50 L of SOT media (NaHCO₃ 16.8 g/L, K₂HPO₄ 0.5 g/L, NaNO₃ 2.5 g/L, K₂SO₄ 1g/L, NaCl 1g/L, MgSO₄·7H₂O 0.2 g/L, CaCl₂·2H₂O 0.04 g/L, FeSO₄·7H₂O 0.01 g/L, EDTA 0.08 g/L, Trace Metal Mix A5(H₃BO₃ 2.86 g/L, MnCl₂·4H₂O 1.81 g/L, ZnSO₄·7H₂O 0.222 g/L, Na₂MoO₄·2H₂O 0.39 g/L, CuSO₄·5H₂O 0.079 g/L, Co(NO₃)₂·6H₂O 49.4 mg/L) 1.0 ml/L, Trace Metal Mix B6 Modified(NH₄NO₃ 0.23 g/L, K₂Cr₂(SO₄)₄·24H₂O 96 mg/L, NiSO₄·7H₂O 47.8 mg/L, Na₂WO₄·2H₂O 17.9 mg/L, Ti₂(SO₄)₃40 mg/L) 1.0 ml/L and a proper quantity of NaOH, pH 9.5) inoculated with Spirulina (*S. platensis* ATCC 53843). After termination of the primary culture, 50 L of fresh SOT media was provided to the culture apparatus and a further culture was performed for 20 days under the same condition with the primary culture. When the further culture was terminated, the final culture solution was harvested and algal bodies were isolated via a filtration. After drying, the dry weight of the algal biomass was measured. In addition, the inner surface area to which the algae bodies were attached and the ratio of the area to total inner surface area of the culture apparatus was measured.

### Comparative example 2: Cultivation of Spirulina using a prior culture apparatus for microalgae

The present inventors cultivated Spirulina using a culture apparatus for microalgae using disclosed in Korean Patent Gazette No. 2004-0019298.

Particularly, the present inventors performed a primary culture for 3 days using the culture apparatus at 35 °C under 6,000 lux of illumination after adding 50 L of SOT media inoculated with Spirulina (*S. platensis* ATCC 53843) between an inner chamber and an outer chamber via an opening part of the apparatus and then injecting 1:1 (v/v) CO₂/air mixed gases continuously via an opening of the apparatus and making a spiral flow. After termination of the primary culture, 50 L of fresh SOT media was provided to the culture apparatus and a further culture was performed for 20 days under the same condition with the primary culture. When the further culture was terminated, the final culture solution was harvested from the opening part and algal bodies were isolated via a filtration. After drying, the dry weight of the algal biomass was measured. In addition, the inner surface area of the outer chamber to which the algae bodies were attached and the ratio of the area to total inner surface area of the outer chamber was measured.

### Example 1: Cultivation of Spirulina using a tubular cultural apparatus for microalgae

The present inventors performed cultivation in a circulatory way using a novel culture apparatus for microalgae depicted in Figs. 1 to 3.

Above all, liquid spawn of *S. platensis* ATCC 53843 was inoculated in SOT media at the ration of 1:10 (v/v). Subsequently a first culture solution inlet 131, a first culture solution outlet 132 and a final outlet 133 were closed and 50 L of SOT media inoculated with Spirulina was injected into a culture tank 101 via an inoculum inlet 110. And then, a primary culture was performed at 35°C controlled by a temperature controller 160 under 6,000 lux of illumination which is provided by a first light source with the continuously injection of CO₂ and with stirring at 60 rpm using an agitator 150. At this time, optical density at 680 nm (OD₆₈₀) was detected using a spectrophotometer (Ultraspec™ 3100 Pro, Amersham, USA) in order to measure the growth rate of cultivated Spirulina.

After termination of the primary culture, 50 L of fresh SOT media was provided to the culture tank 101 and mixed gases of carbon dioxide and nitrogen was injected continuously via a gas inlet 111 of a first cultivating part in order to provide carbon dioxide used for photosynthesis and to apply positive pressure of about 1.0 kg/cM²f (100 kPa) to the culture tank 101 for preventing contamination by various germs. And then, the culture solution was mixed with the fresh media by operating an agitator 150 with a constant speed and the first culture solution inlet 131 and the first culture solution outlet 132 were opened. Accordingly, the mixed culture solution was transferred to a third culture solution inlet 210 of a pump part 200. When the mixed culture solution was transferred to the third culture solution inlet 210 of the pump part 200, a pump 220 of the pump part was operated in order that the mixed culture solution could be transferred to a second culture solution inlet 310 through a third culture solution outlet 230 at a rate of 1 m/sec. The overall flow rate in the culture pipe 330 was controlled to 25 cm/sec. The mixed culture solution transferred to the second culture solution inlet 310 was transferred to a culture pipe 330, a second culture solution outlet 340 and the first culture solution inlet 131, sequentially. Finally the mixed culture solution was transferred to the culture tank 101 via the first culture solution inlet 131 making a rotation in the circulation. At this time, the further culture along with photosynthesis was performed by illuminating 6,000 lux of illumination using a second light source coupled to the culture pipe 330 and temperature was maintained to 35°C via the temperature controller 160 for 20 days. After termination of the further culture, the final culture solution was harvested from the final outlet 133 of the first cultivating part 100 and biomass of Spirulina was isolated by filtration.

After drying the biomass, the dry weight of the biomass was measured. In addition, the inner surface area of the culture pipe 330 to which the algae bodies were attached and the ratio of the area to total inner surface area of the culture pipe 330 was measured. The dry weight of the biomass and the ratio were compared to those of the comparative examples 1 and 2 (Table 1).

**Table 1: The dry weight of biomass of Spirulina and the ratio of the attached area to total inner surface area of the culture vessels**

| Examples | Dry weight of biomass of Spirulina | The ratio of the attached area to total inner surface area of the culture vessels |
|---|---|---|
| Example 1 | 25 kg | 3.4% |
| Comparative example 1 | 8 kg | 87% |
| Comparative example 2 | 12 kg | 67% |

As shown in Table 1, using the circulatory method for cultivating photosynthetic microalgae, the dry weight of microalgal biomass was increased 2 to 3 times more than those of using prior culture apparatus. This is due to prevention of attachment of microalgae to the inner surface of culture vessels when a circulatory photobioreactor is used. Indeed, when the circulatory cultivating method of the present invention was used, only about 3.4% of total inner surface area of culture vessel was occupied with attached Spirulina, whereas 67% and 87% of total inner surface of culture vessels were occupied with attached Spirulina when prior culture apparatuses were used.

Spirulina is a kind of photosynthetic bacteria and the growth rate in varied according to amount of illumination. Thus, if algal bodies of Spirulina are attached to the inner surface of culture vessels, the efficiency of the growth is reduced. Accordingly, it was confirmed that the productivity of photosynthetic microalgae including Spirulina can be improved by using the circulatory method for cultivating photosynthetic microalgae and by preventing attachment of photosynthetic microalgal bodies to the inner surface of culture vessels thereby.

## Claims

1. A circulatory method for cultivating photosynthetic microalgae, the method comprising:
a) injecting culture media in which photosynthetic microalgae, preferably genus *Spirulina* is inoculated into a first cultivating part of a photobioreactor and performing a primary culture by illuminating the first cultivating part;
b) mixing culture solution after terminating the primary culture with fresh media added to the cultivating part and then transferring the mixed culture solution to a second cultivating part connected to the first cultivating part;
c) performing a further culture by illuminating the second cultivating part;
d) circulating further cultivated culture solution from the second cultivating part to the first cultivating part; and
e) recovering the culture solution and then filtering and harvesting photosynthetic microalgal bodies after terminating the cultivating.

2. The method according to claim 1, wherein the first culture and/or the further culture are performed under 4,000 to 8,000 lux of illumination.

3. The method according to claims 1 or 2, wherein the first culture and/or the further culture are performed at the temperature of 32 to 38°C.

4. The method according to any one of claims 1 to 3, wherein the first culture and/or the further culture are performed at pH of 8.5 to 10.

5. The method according to any one of claims 1 to 4, wherein the first cultivating part comprises a culture tank and transfer and circulation between the first cultivating part and the second cultivating part are performed by a pump.

6. The method according to any one of claims 1 to 5, wherein photosynthesis by the photosynthetic microalgae is performed by injecting mixed gases of carbon dioxide and nitrogen through the first cultivating part and wherein contamination by various germs is prevented by maintaining positive pressure within the first cultivating part during the primary culture, preferably 0.1 to 1.0 kg/cM²f (10-100 kPa).

7. The method according to any one of claims 1 to 6, wherein the circulating of step d is performed at a flow rate of 1 to 50 cm/s.

8. A circulatory method for cultivating photosynthetic microalgae, the method comprising:
a) performing a primary culture by injecting culture media comprising an inoculum of photosynthetic microalgae, preferably genus *Spirulina* to the first cultivating part and illuminating the first cultivating part;
b) mixing culture solution after terminating the primary culture with fresh media added to the cultivating part and circulating the mixed culture solution to the second cultivating part and to the first cultivating part, and performing a further culture by illuminating the second cultivating part; and
c) harvesting the photosynthetic microalgae by recovering and filtering the culture solution after the further culture.

9. The method according to claim 8, wherein the first culture and/or the further culture are performed under 4,000 to 8,000 lux of illumination.

10. The method according to claims 8 or 9, wherein the first culture and/or the further culture are performed at the temperature of 32 to 38°C.

11. The method according to any one of claims 8 to 10, wherein the first culture and/or the further culture are performed at pH of 8.5 to 10.

12. The method according to any one of claims 8 to 11, wherein the first cultivating part comprises a culture tank and transfer and circulation between the first cultivating part and the second cultivating part is performed by a pump.

13. The method according to any one of claims 8 to 12, wherein a photosynthesis of the photosynthetic microalgae is performed by injecting mixed gases of carbon dioxide and nitrogen through the first cultivating part and wherein contamination of various germs is prevented by maintaining positive pressure within the first cultivating part during the primary culture, preferably 0.1 to 1.0 kg/cm²f (10-100 kPa).

14. The method according to any one of claims 8 to 13, wherein the circulating of step b is performed at a flow rate of 1 to 50 cm/s.
